# EUROPEAN PATENT SPECIFICATION

(11) **EP 4 312 876 B1**
(45) Date of publication and mention of the grant of the patent: **28.10.2026**
(21) Application number: 22715959.7
(22) Date of filing: 23.03.2022
(51) Int. Cl.: A61F 2/00, A61F 2/26, A61F 2/48, F04B 43/04, F04B 23/02

(54) **PUMP ASSEMBLY FOR AN IMPLANTABLE INFLATABLE DEVICE**
PUMPENANORDNUNG FÜR EINE IMPLANTIERBARE AUFBLASBARE VORRICHTUNG
ENSEMBLE POMPE POUR DISPOSITIF GONFLABLE IMPLANTABLE

(30) Priority: 25.03.2021 US 202163200737 P; 22.03.2022 US 202217655937
(43) Date of publication of application: 07.02.2024
(73) Proprietor: Boston Scientific Scimed, Inc., Maple Grove, MN 55311-1566 (US)
(72) Inventor: SMITH, Noel, Kilkenny, R95 HX88 (IE); MAILE, Keith R., New Brighton, Minnesota 55112 (US); ALBRECHT, Thomas Andrew, Edina, Minnesota 55410 (US); NOLAN, Daragh, Waterford, P36 TC98 (IE); WATSCHKE, Brian P., Minneapolis, Minnesota 55405 (US); SINNOTT, Thomas, Enniscorthy Wexford (IE); PERCY, Richard, Cork (IE); JOHNSON, Bryan Duane, Mahtomedi, Minnesota 55115 (US); NORRIS, Laurence, Waterford (IE); GILDEA, John, Kildare, W23F40T (IE); MARCOS LARANGEIRA, Eduardo, Clonmel Tipperary, E91 W1R9 (IE)
(74) Representative: Pfenning, Meinig & Partner mbB
(86) International application number: PCT/US2022/071294
(87) International publication number: WO 2022/204698

(56) References cited:
- EP-A2- 2 712 600
- WO-A2-2009/094431
- US-A1- 2019 133 737
- US-A1- 2019 350 712
- US-A1- 2020 222 188

## Description

### TECHNICAL FIELD

This disclosure relates generally to bodily implants, and more specifically to bodily implants including a pump.

### BACKGROUND

Active implantable fluid operated inflatable devices often include one or more pumps that regulate a flow of fluid between different portions of the implantable device to provide for inflation and deflation of one or more fluid fillable implant components of the device. One or more valves can be positioned within fluid passageways of the device to direct and control the flow of fluid so as to achieve inflation, deflation, pressurization, depressurization, activation, deactivation and the like of the different fluid fillable implant components of the device. In some implantable fluid operated inflatable devices, sensors can be used to monitor fluid pressure and/or fluid volume and/or fluid flow rate within fluid passageways of the device. Accurate monitoring of conditions within the device, including pressure monitoring and flow monitoring, may provide for improved control of device operation, improved diagnostics, and improved efficacy of the device.

US 2019/350712 A1 relates to an inflatable penile prosthesis including a fluid reservoir configured to hold fluid, an inflatable member, and a pump assembly configured to transfer the fluid from the fluid reservoir to the inflatable member during an inflation cycle. The pump assembly includes a first pump configured to inject the fluid into the inflatable member according to a first flow rate, and a second pump configured to inject fluid into the inflatable member according to a second flow rate, where the second flow rate is less than the first flow rate.

Further examples of inflatable implantable devices are provided in WO 2009/094431 A2, US 2019/133737 A1, US 2020/222188 A1 and EP 2 712 600 A2.

### SUMMARY

The invention is set out in the appended set of claims. It relates to an implantable fluid operated inflatable device as defined in claim 1.

According to an aspect, an implantable fluid operated inflatable device includes a fluid reservoir; an inflatable member; and a pump and valve assembly configured to transfer fluid between the fluid reservoir and the inflatable member. The pump assembly includes a manifold, including a housing; at least one valve and at least one pump positioned in a fluid passageway formed in the housing; a first fluid port in fluidic communication with the fluid reservoir; and a second fluid port in fluidic communication with the inflatable member. The device also includes an electronic control system controlling operation of the pump and valve assembly; and at least one pressure sensing device in communication with the electronic control system.

The at least one valve and the at least one pump includes a first pump and a first valve positioned in a first fluid passageway and in fluidic communication with the first fluid port; and a second pump and a second valve positioned in a second fluid passageway and in fluidic communication with the second fluid port. The at least one pressure sensing device can include a first pressure sensing device positioned in the first fluid passageway and configured to measure a pressure of fluid flowing through the first fluid port and to transmit the measured pressure to the electronic control system; and a second pressure sensing device positioned in the second fluid passageway and configured to measure a pressure of fluid flowing through the second fluid port and to transmit the measured pressure to the electronic control system.

In some implementations, the at least one valve and the at least one pump includes a dual piezoelectric pump manifold configuration, including a first piezoelectric pump; a second piezoelectric pump; and a fluid channel providing for fluidic communication between the first piezoelectric pump and the second piezoelectric pump. The first piezoelectric pump can include a first chamber; a first piezoelectric diaphragm positioned along an edge portion of the first chamber; a first check valve at an inlet end of the first chamber; and a second check valve at an outlet end of the first chamber, the second check valve of the first piezoelectric pump selectively providing fluidic communication between the first chamber and the fluid channel. The second piezoelectric pump can include a second chamber; a second piezoelectric diaphragm positioned along an edge portion of the second chamber; a first check valve at an inlet end of the second chamber, the first check valve of the second piezoelectric pump selectively providing fluidic communication between the fluid channel and the second chamber; and a second check valve at an outlet end of the second chamber. In some implementations, a pumping cycle of the dual piezoelectric pump manifold configuration includes a first phase including a supply stroke of the first piezoelectric diaphragm in coordination with a pressure stroke of the second piezoelectric diaphragm; and a second phase including a pressure stroke of the first piezoelectric diaphragm in coordination with a supply stroke of the second piezoelectric diaphragm. In some implementations, in the first phase, fluid is drawn into the first chamber through the first check valve of the first piezoelectric pump, and fluid is expelled from the second chamber through the second check valve of the second piezoelectric pump; and in the second phase, fluid is expelled from the first chamber and into the fluid channel through the second check valve of the first piezoelectric pump, and fluid is drawn from the fluid channel into the second chamber through the first check valve of the second piezoelectric pump.

In some implementations, the housing of the manifold is made of an injection molded metal material, machined metal material and the like, with the at least one pump and the at least one valve positioned in a sealed fluid passageway defined in the injection molded metal material, such that the manifold is a hermetic manifold.

In some implementations, the pump assembly includes a pump assembly housing, and wherein the manifold and the electronic control system are received in the pump assembly housing. The manifold can be a hermetic manifold, such that components of the electronic control system within the pump assembly housing are isolated from fluid flowing through the hermetic manifold.

In some implementations, the at least one pressure sensing device includes a first pressure sensing device positioned proximate a fluid port of the reservoir; and a second pressure sensing device positioned proximate a fluid port of the inflatable member. The first pressure sensing device can include a first diaphragm positioned in a fluid passageway proximate the reservoir, facing the reservoir; and at least one first strain gauge mounted on the first diaphragm, the at least one first strain gauge being configured to measure a deflection of the first diaphragm and to transmit the measured deflection to the electronic control system. The second pressure sensing device can include a second diaphragm positioned in a fluid passageway proximate the fluid port of the inflatable member, facing the inflatable member; and at least one second strain gauge mounted on the second diaphragm, the at least one second strain gauge being configured to measure a deflection of the second diaphragm and to transmit the measured deflection to the electronic control system.

In some implementations, the at least one sensing device includes at least one piezoelectric element positioned in a fluid passageway of the implantable fluid operated device and configured to sense a fluid pressure level in the fluid passageway based on an input voltage level applied to the piezoelectric element and an output voltage level measured at the piezoelectric element.

In some implementations, the electronic control system includes a printed circuit board including a processor configured to receive pressure level measurements from the at least one sensing device; apply a control algorithm based on the received pressure level measurements; and control operation of the at least one valve and the at least one pump in accordance with the applied control algorithm.

In some implementations, the implantable fluid operated device is an artificial urinary sphincter or an inflatable penile prosthesis.

In another general aspect, an implantable fluid operated inflatable device includes a fluid reservoir; an inflatable member; a pump assembly received in a housing and configured to transfer fluid between the fluid reservoir and the inflatable member, and an electronic control system. The pump assembly can include a manifold; and a pump and valve device received in the manifold. The electronic control system can be configured to control operation of the pump and valve device.

In some implementations, the manifold is a hermetic manifold, and the electronic control system includes a first portion received in an electronics compartment of the housing, isolated from fluid flowing through the manifold. In some implementations, the electronic control system includes a second portion that is external to the implantable fluid operated inflatable device, and is configured to communicate with of the first portion of the electronic control system, wherein the second portion is configured to receive user inputs, and to output information to the user.

In some implementations, the pump and valve device is a dual piezoelectric pump and valve configuration device, including a first piezoelectric pump in fluidic communication with a second piezoelectric pump via a fluid channel in a manifold or housing. The first piezoelectric pump can include a first chamber; a first piezoelectric element and diaphragm positioned along an edge portion of the first chamber; a first check valve at an inlet end of the first chamber; and a second check valve at an outlet end of the first chamber, the second check valve of the first piezoelectric pump selectively providing fluidic communication between the first chamber and the fluid channel. The second piezoelectric pump can include a second chamber; a second piezoelectric element and diaphragm positioned along an edge portion of the second chamber; a first check valve at an inlet end of the second chamber, the first check valve of the second piezoelectric pump selectively providing fluidic communication between the fluid channel and the second chamber; and a second check valve at an outlet end of the second chamber. In some implementations, in an inflation mode, the electronic control system is configured to alternately apply a voltage input to the first piezoelectric element and the second piezoelectric element to cause fluid to flow through the dual piezoelectric pump manifold configuration in a first direction, from the fluid reservoir toward the inflatable member; and in a deflation mode, the electronic control system is configured to alternately apply a voltage input to the first piezoelectric element and the second piezoelectric element to cause fluid to flow through the dual piezoelectric pump manifold configuration in a second direction, from the inflatable member toward the reservoir.

### BRIEF DESCRIPTION OF THE DRAWINGS

FIG. 1 is a block diagram of an implantable fluid operated inflatable device according to an aspect.
FIGS. 2A and 2B illustrate example implantable fluid operated inflatable devices according to an aspect.
FIG. 3 is a schematic diagram of a fluid architecture of a pump assembly of an implantable fluid operated inflatable device according to an aspect.
FIGS. 4A and 4B are perspective views of an example manifold of an example pump assembly according to an aspect.
FIGS. 5A and 5B are perspective views of the example manifold installed in an example pump assembly of an implantable fluid operated inflatable device according to an aspect.
FIGS. 6A-6C schematically illustrate operation of an example piezoelectric pump of an implantable fluid operated inflatable device according to an aspect.
FIGS. 7A-7C schematically illustrate operation of an example dual piezoelectric pump & valve manifold configuration of an implantable fluid operated inflatable device according to an aspect.
FIG. 8 is a block diagram of operation of an example dual piezoelectric pump & valve manifold configuration of an implantable fluid operated inflatable device according to an aspect.
FIGS. 9A and 9B are schematic views of implantable fluid operated inflatable devices including inline pressure sensing devices according to an aspect.
FIGS. 10A-10C are graphs illustrating the effect of changes in atmospheric pressure on measured pressure in an implantable fluid operated inflatable device.
FIGS. 11A-11D are graphs illustrating the effect of an impulse at an inflatable member on measured pressure in an implantable fluid operated inflatable device.
FIGS. 12A-12D are graphs illustrating the effect of an impulse at a reservoir on measured pressure in an implantable fluid operated inflatable device.
FIGS. 13A-13D are graphs illustrating the effect of a component failure or blockage in a fluid passageway on measured pressure in an implantable fluid operated inflatable device.
FIG. 14A is a top view, and FIGS. 14B and 14C are side views, of an example diaphragm fitted with strain gauges for measurement of deflection of the diaphragm.

### DETAILED DESCRIPTION

Detailed implementations are disclosed herein. However, it is understood that the disclosed implementations are merely examples, which may be embodied in various forms. Therefore, specific structural and functional details disclosed herein are not to be interpreted as limiting, but merely as a basis for the claims and as a representative basis for teaching one skilled in the art to variously employ the implementations in virtually any appropriately detailed structure. Further, the terms and phrases used herein are not intended to be limiting, but to provide an understandable description of the present disclosure.

The terms "a" or "an," as used herein, are defined as one or more than one. The term "another," as used herein, is defined as at least a second or more. The terms "including" and/or "having", as used herein, are defined as comprising (i.e., open transition). The term "coupled" or "moveably coupled," as used herein, is defined as connected, although not necessarily directly and mechanically.

In general, the implementations are directed to bodily implants. The term patient or user may hereinafter be used for a person who benefits from the medical device or the methods disclosed in the present disclosure. For example, the patient can be a person whose body is implanted with the medical device or the method disclosed for operating the medical device by the present disclosure.

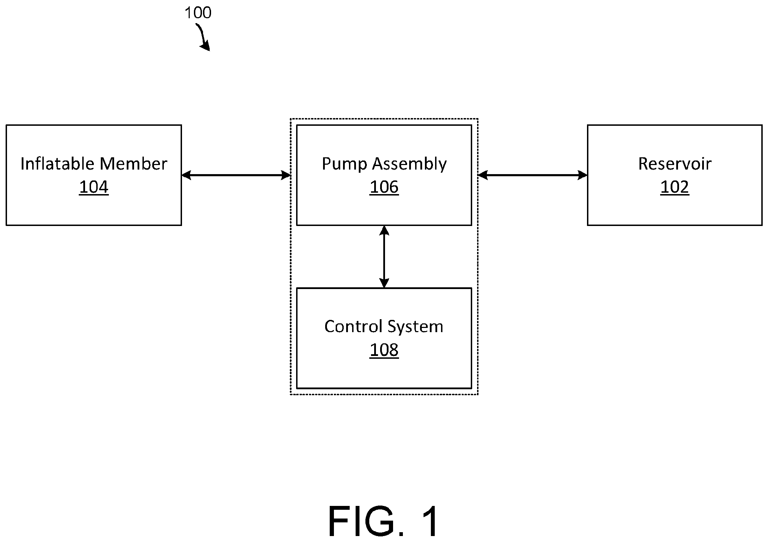

FIG. 1 is a block diagram of an example implantable fluid operated inflatable device 100. The example device 100 shown in FIG. 1 includes a fluid reservoir 102, an inflatable member 104, and a pump assembly 106 configured to transfer fluid between the fluid reservoir 102 and the inflatable member 104. In some implementations, the example device 100 includes a control system 108. In some implementations, the control system 108 is an electronic control system 108. The control system 108 may provide for the monitoring and/or control of the operation of various components of the pump assembly 106 and/or communication with one or more sensing device(s) within the implantable fluid operated inflatable device 100 and/or communication with one or more external device(s). The fluid reservoir 102, the inflatable member 104, and the pump assembly 106 may be internally implanted into the body of the patient. In some implementations, the control system 108 is coupled to or incorporated into the pump assembly 106. In some implementations, at least a portion of the control system 108 is separate or spaced from the pump assembly 106. In some implementations, some modules of the control system 108 are coupled to or incorporated into the pump assembly 106, and some modules of the control system 108 are separate from the pump assembly 106. For example, in some implementations, some modules of the control system 108 are included in an external device that is in communication other modules of the control system 108 included within the implanted device 100. In some implementations, the pump assembly 106 is electronically controlled. In some implementations, the pump assembly 106 is manually controlled.

In some examples, electronic monitoring and control of the fluid operated device 100 may provide for improved patient control of the device, improved patient comfort, and improved patient safety. In some examples, electronic monitoring and control of the fluid operated device 100 may afford the opportunity for tailoring of the operation of the device 100 by the physician without further surgical intervention.

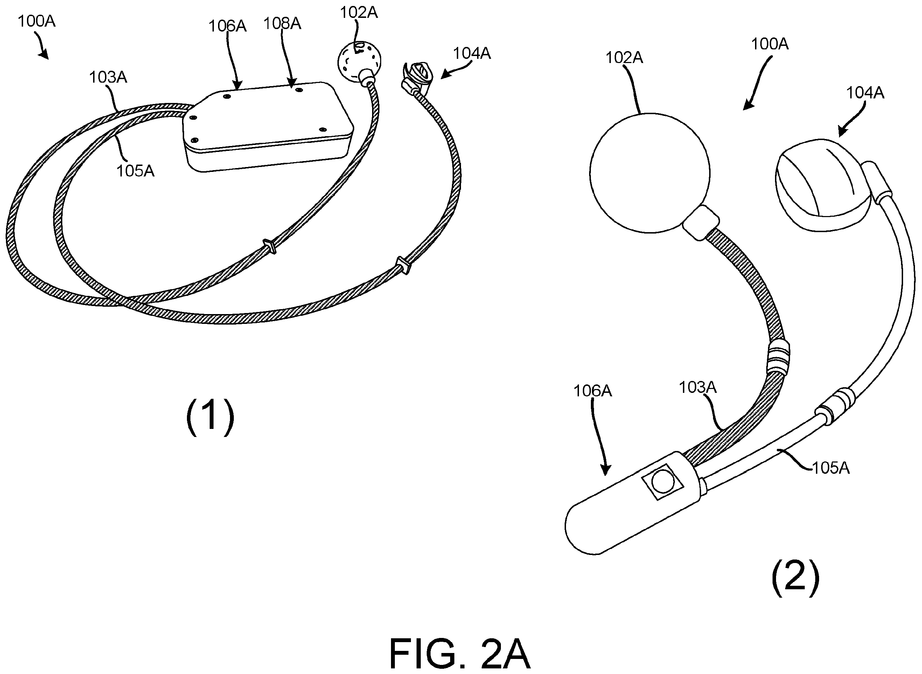

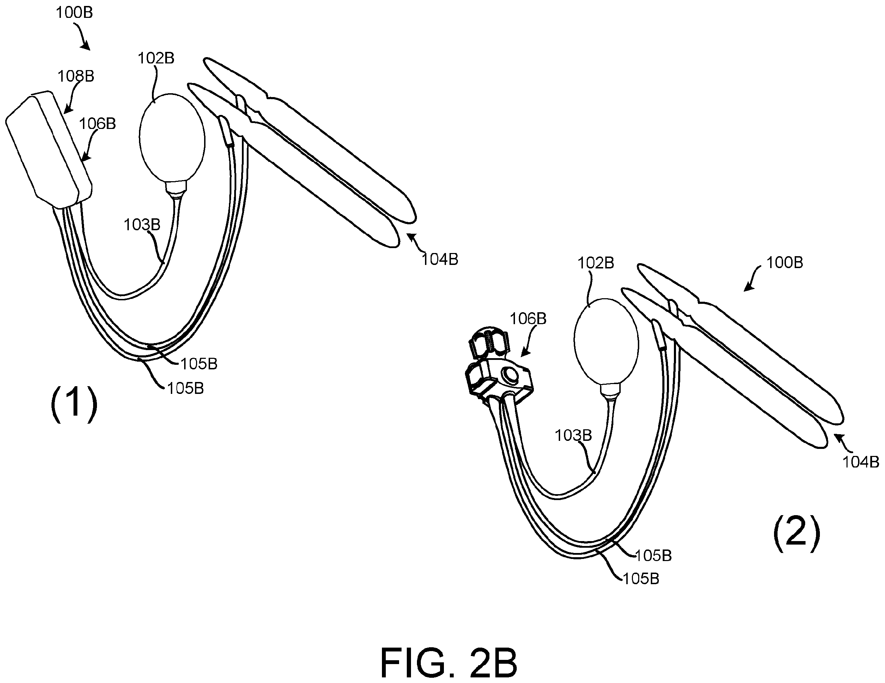

The example implantable fluid operated device 100 may be representative of a number of different types of implantable fluid operated devices. For example, the device 100 shown in FIG. 1 may be representative of an artificial urinary sphincter 100A as shown in FIG. 2A. The example artificial urinary sphincter 100A includes a pump assembly 106A. In the example shown in FIG. 2A(1), a control system 108A controls, for example, electronically controls, operation of the pump assembly 106A to provide for the transfer of fluid between a reservoir 102A and an inflatable cuff 104A. In the example shown in FIG. 2B, the pump assembly 106A may be manually controlled. A first conduit 103A connects the pump assembly 106A/control system 108A with the reservoir 102A. A second conduit 105A connects the pump assembly 106A/control system 108A with the inflatable cuff 104A. In some examples, the device 100 shown in FIG. 1 may be representative of an inflatable penile prosthesis 100B as shown in FIG. 2B. The example penile prosthesis 100B includes a pump assembly 106B. In the example shown in FIG. 2B(1), a control system 108B controls, for example, electronically controls, operation of the pump assembly 106A to provide for the transfer of fluid between a fluid reservoir 102B and inflatable cylinders 104B. In the example shown in FIG. 2B(2), the pump assembly 106B may be manually controlled. A first conduit 103B connects the pump assembly 106B/control system 108B with the reservoir 102B. One or more second conduits 105B connect the pump assembly 106A/control system 108A with the inflatable cylinders 104B. The principles to be described herein may be applied to these and other types of implantable fluid devices that rely on a pump assembly to provide for the transfer of fluid between the different fluid filled implant components to achieve inflation, deflation, pressurization, depressurization, deactivation and the like for effective operation. The example devices 100A, 100B may include electronic control systems 108A, 108B to provide for the monitoring and control of pressure and/or fluid flow through the respective devices 100A, 100B. The principles to be described herein may also be applied to implantable fluid operated devices that are manually controlled.

As noted above with respect to FIG. 1, the pump assembly can include one or more pumps and one or more valves positioned within a fluid circuit of the pump assembly to control the transfer fluid between the fluid reservoir and the inflatable member. In some examples, the pump(s) and/or the valve(s) are electronically controlled. In some examples, the pump(s) and/or the valve(s) are manually controlled. In some examples, the pump assembly includes a fluid manifold having fluidic channels formed therein, defining the fluid circuit. In an example in which the pump assembly is electronically powered and/or controlled, the manifold may be a hermetic manifold that can contain and segment the flow of fluid from electronic components of the pump assembly, to prevent leakage and/or gas exchange. In some examples, the pump assembly includes one or more pressure sensing devices in the fluid circuit to provide for relatively precise monitoring and control of fluid flow and/or fluid pressure within the fluid circuit and/or the inflatable member. A fluid circuit configured in this manner may facilitate the proper inflation, deflation, pressurization, depressurization, activation and deactivation of the components of the implantable fluid operated device to provide for patient safety and device efficacy.

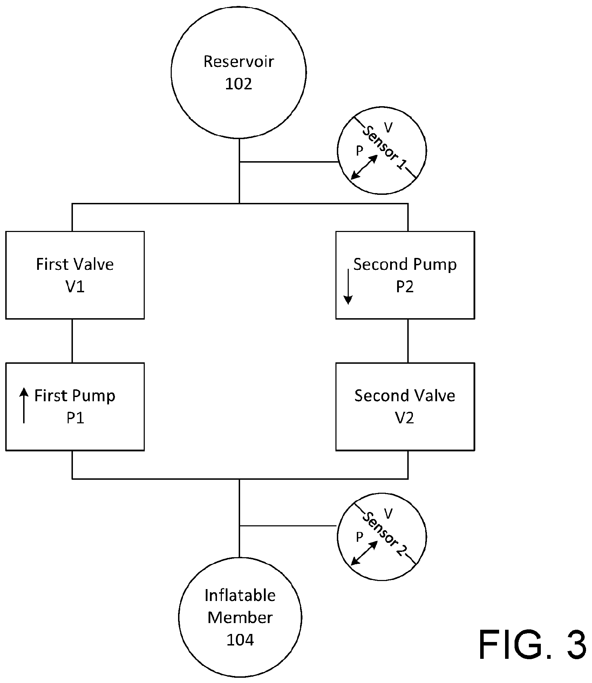

FIG. 3 is a schematic diagram of an example fluidic architecture for an implantable fluid operated device, according to an aspect. The schematic diagram shown in FIG. 3 is just one example arrangement. The fluidic architecture of an implantable fluid operated device can include other orientations of fluidic channels, valve(s), pressure sensor(s) and other components. A fluidic architecture that can accommodate back pressure, pressure surges and the like enhances the performance, efficacy and efficiency of the fluid operated device 100.

The example fluidic architecture shown in FIG. 3 includes channels guiding the flow of fluid between the reservoir 102 and the inflatable member 104. In the example shown in FIG. 3, a first valve V1 in a first fluidic channel controls the flow of fluid, generated by a first pumping device P1, from the inflatable member 104 to the reservoir 102. A second valve V2 in a second fluidic channel controls the flow of fluid, generated by a second pumping device P2, from the reservoir 102 to the inflatable member 104. In the example shown in FIG. 3, a first pressure sensing device S1 senses a fluid pressure at the reservoir 102, and a second pressure sensing device S2 senses a fluid pressure at the inflatable member 104. The first and second pressure sensing devices S1, S2 may provide for the monitoring of fluid flow and/or fluid pressure in the fluidic channels. In the arrangement shown in FIG. 3A, one of the first pump P1 or the second pump P2 is active, while the other of the first pump P1 or the second pump P2 is in a standby mode, such that the first and second pumps do not typically operate simultaneously. For example, operation of the first pump P1 (with the second pump P2 in the standby mode) may provide for the deflation of the inflatable member 104, and operation of the second pump P2 (with the first pump P1 in the standby mode) may provide for the inflation of the inflatable member 104. The valves V1, V2 may provide for the selective sealing of the respective fluidic channel(s) so as to maintain a set state of the fluid operated device. In some implementations, the valves V1, V2 may facilitate the transition between states (i.e., inflated and deflated states) of the fluid operated device. For example, selective sealing of the respective fluidic channel(s) by the valves V1, V2 may maintain an inflated state or a deflated state of the inflatable member 104. Interaction with the valves V1, V2 (and the corresponding change in fluid flow through the fluidic architecture of the device) may change the set state of the fluid operated device. Valves V1, V2 that maintain the set state of the device until the patient requires a change in the set state of the device and initiates the required change in the set state of the device provide enhanced patient safety and improved device efficacy.

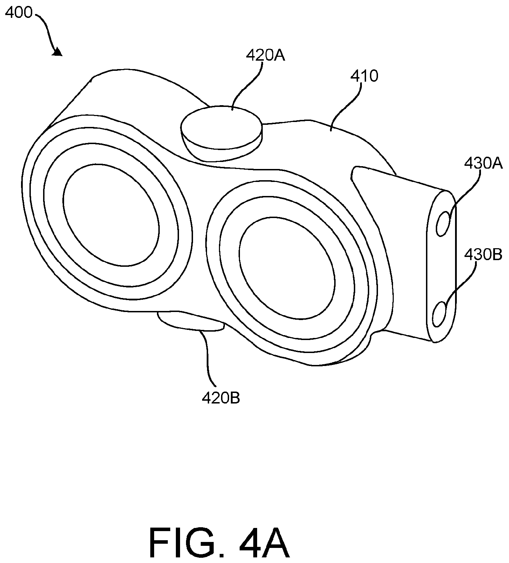

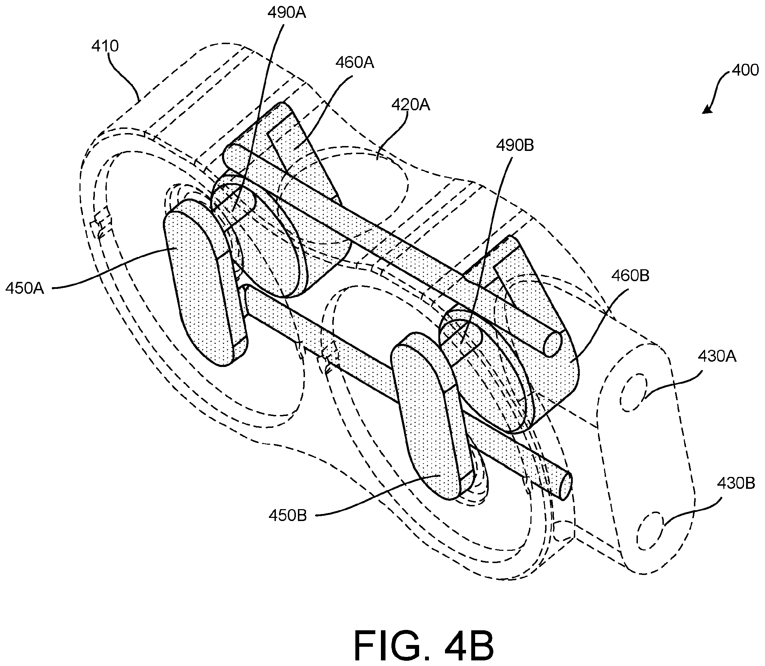

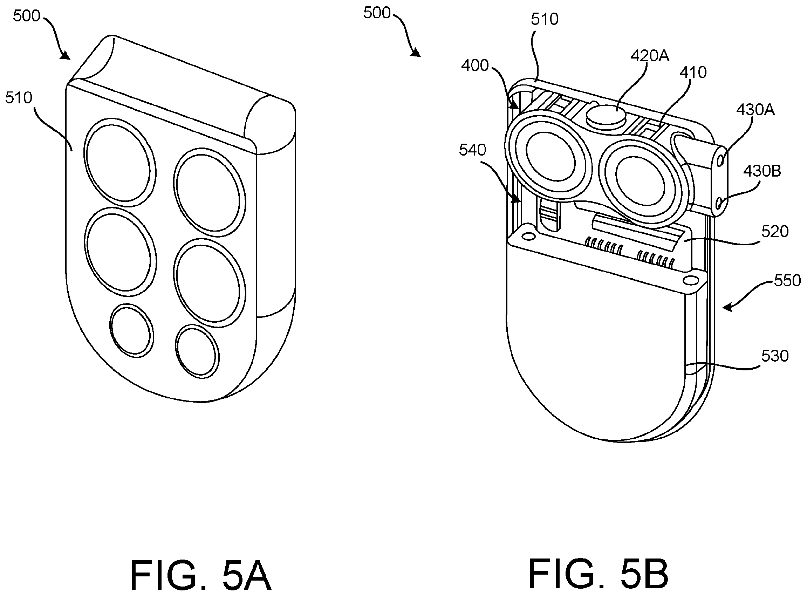

FIGS. 4A and 4B are perspective views of an example manifold 400 for use with a pumping assembly of an implantable fluid operated device. In FIG. 4B, a housing 410 of the example manifold 400 is transparent, so that an arrangement of internal fluidics components (valve(s), pump(s), sensor(s) and the like) of the manifold 400 is visible. FIGS. 5A and 5B are perspective views of an example pump assembly 500 including the manifold 400 and an electronic control system 550. In FIG. 5B, a portion of a housing 510 of the pump assembly 500 has been removed so that internal components of the pump assembly 500 are visible.

The example manifold 400 may employ a fluidic architecture such as the fluidic circuit defined by the schematic diagram shown in FIG. 3, or other fluidic architecture. The fluidic architecture of the manifold 400 may provide for the controlled transfer and monitoring of fluid in an implantable fluid operated device (such as the example devices 100 illustrated in FIGS. 2A and 2B), between the fluid reservoir 102 and the inflatable member 104.

The manifold 400 may include a housing 410. Fluid passageways may be defined within the housing 410, with fluidics components positioned within the fluid passageways. In some examples, the housing 410 may be manufactured from a solid piece of material. In some examples, the housing 410 may be molded, for example, injection molded. In some examples, the housing 410 is made of a metal material such as, for example, titanium, steel, or other biocompatible material. This may allow fluidics components to be installed in fluid passageways defined within the housing 410, and the fluid passageways to be sealed. The manifold 400/housing 410 manufactured in this manner may be hermetic, such that fluids flowing through the manifold 400 and components received in the manifold 400 are contained within the manifold 400. In a situation in which one or more of the fluidics components includes a non-biocompatible material, the hermetic nature of the manifold 400 may prevent leaching of these materials into the body of the patient, thus improving patient safety considerations.

According to the invention that is shown in FIG. 4B, the manifold 400 includes a first pump 450A in fluidic communication with a first valve 460A via a first fluid passageway 490A, and a second pump 450B in fluidic communication with a second valve 460B via a second fluid passageway 490B. The first pump 450A and the first valve 460A may direct fluid out of the manifold 400 through a first outlet port 430A to the reservoir 102 of the fluid operated device 100. The second pump 450B and the second valve 460B may direct fluid out of the manifold 400 through a second outlet port 430B to the inflatable member 104 of the fluid operated device 100. A first pressure sensing device 420A senses a fluid pressure of fluid flowing between the manifold 400 and the reservoir 102. A second pressure sensing device 420B senses a fluid pressure of fluid flowing between the manifold 400 and the inflatable member 104.

In some examples, the first valve 460A and/or the second valve 460B are normally open valves. In an arrangement in which the first and second valves 460A, 460B are normally open valves, the second valve 460B may be actuated to cause the second valve 460B to close while the first pump 450A operates to cause fluid to flow from the manifold 400 to the reservoir 102. Similarly, the first valve 460A may be actuated to cause the first valve 460A to close while the second pump 450B operates to cause fluid to flow from the manifold 400 to the inflatable member 104. Normally open valves may enhance patient safety considerations, for example, providing for the relief of pressure at the inflatable member 104 in the event of faults, failures, blockages and the like within the fluidics architecture.

As discussed above, in some examples, control system components are incorporated into the pump assembly 500, to control and monitor operation of the pump assembly 500, and/or to provide for communication with external device(s). For example, as shown in FIGS. 5A and 5B, an electronic control system 550 may be incorporated into the pump assembly 500, together with the fluidics architecture and components in the manifold 400. FIG. 5A illustrates a stacked arrangement of components in the manifold 400. FIG. 5B illustrates a vertical arrangement of components in the manifold 400. The electronic control system 550 may include, for example, a printed circuit board (PCB) 520, a power storage device 530, battery 530, and other such electronic components. In some examples, the PCB 520 may include a processor providing processing capability, a memory, a communications module providing for communication with other electronic components, sensors and the like, as well as communication with external devices, control functionality providing for control of operation of the device, and the like. In some examples, the PCB 520 provides for the processing of inputs such as pressure and/or fluid flow measurements received from sensors of the device, the application of control algorithms to the received inputs, and the output of control functionality based on the application of the algorithms. The electronic components may be received in an electronics compartment 540 of the pump assembly 500. The electronic components may control operation of the fluidic components received in the fluid passageways in the manifold 400 as described above, may monitor fluid flow volume, fluid pressure and the like at various sections of the flow through the manifold 400 based on information received from the first and second pressure sensing devices 420A, 420B, may communicate with external devices to provide for user control and monitoring of the fluid operated device, and the like. In this type of arrangement, the hermetic manifold 400/housing 410 may isolate fluids flowing through the manifold 400 from electronic components received in the electronics compartment 540. The hermetic nature of the manifold 400 may prevent fluid leakage into the electronics compartment, and may prevent gas exchange between the manifold 400 and the electronics compartment, thus improving reliability, durability and functionality of the device, and further improving patient safety considerations.

As noted above, one or more pressure sensors may be included in the pump assembly for an implantable fluid activated device such as, for example, the devices 100 described above with respect to FIGS. 2A and 2B. In the case of electronically controlled devices, one or more pressure sensors may enable automated regulation of a state of the inflatable member and fluid supplied thereto. The inclusion of one or more pressure sensors also improved diagnostic capabilities, particularly related to isolating fluid flow issues, leakage issues and the like in the fluidic passageways, into and out of the reservoir, into and out of the inflatable member, and the like. Identification of these types of flow related issues provide for early intervention and correction. In some examples, the inclusion of one or more pressure sensors allows for dynamic control of fluid pressure, particularly within the inflatable member, to account for fluctuations due to physical activity. In some examples, the inclusion of one or more pressure sensors provides for the monitoring and control of fluid flow rates. In some examples, pressure sensor(s) included in the pump assembly for an implantable fluid activated device such as, for example, the devices 100 described above with respect to FIGS. 2A and 2B are made of bio-compatible materials, and are relatively compact and power efficient, to provide for monitoring and control of fluid pressure and/or fluid flow through the device, to preserve patient safety with minimal impact on device size and power consumption.

In some examples, the pump assembly includes multiple pressure sensors, as in, for example, the fluidic architecture shown in FIG. 3, which includes two exemplary pressure sensors. In some examples, the pump assembly includes as few as one pressure sensor. In an example including only one pressure sensor, the pressure sensor may be positioned so as to measure pressure at or near the inflatable member. For example, the pressure sensor may be positioned so as to measure fluid pressure in the inflatable member and/or fluid pressure and/or fluid flow into and out of the inflatable member.

In some examples, an electronically controlled pump assembly may provide for measurement of pressure at one or more positions within the pump assembly through the measurement of current at the one or more positions. In some examples, this may be achieved through the placement of a piezoelectric element such as a piezoelectric diaphragm in combination with a passive check valve at the desired position. An increase or a decrease in pressure will affect the deformation of the piezoelectric element. If a deformation of the piezoelectric element (and a corresponding change in voltage) is detected while the piezoelectric pump is not activated, the change in voltage will be indicative of a pressure change, and thus the piezoelectric pump can also function as a pressure sensor.

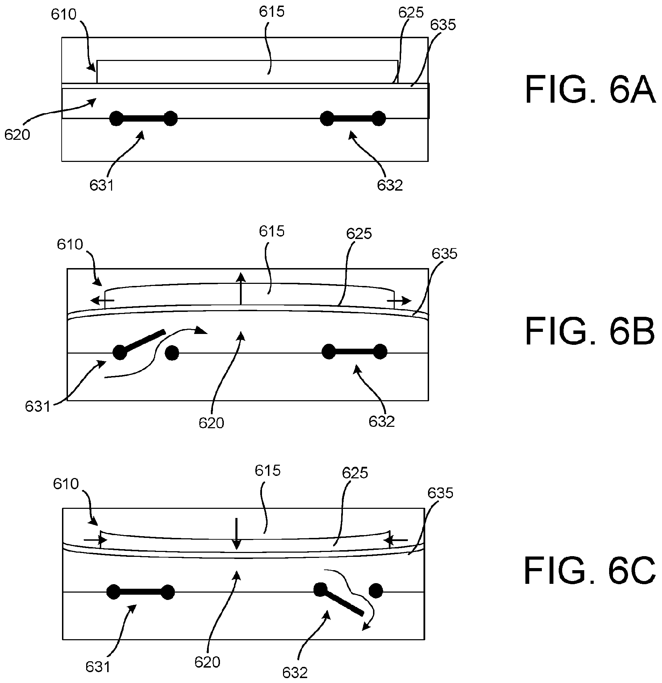

FIG. 6A illustrates a piezoelectric diaphragm 610 positioned in a fluid chamber 620 of a piezoelectric diaphragm pumping device that can provide for the pumping of fluid and also the sensing of pressure. In this example, the piezoelectric diaphragm 610 is positioned along an edge portion of the chamber 620, and includes a single layer disc 615 made of a piezoelectric material (for example, a piezo-ceramic disc) mounted on a plate 625 or membrane 625 attached to an insulative diaphragm 635. A first check valve 631 is positioned at a first side of the chamber 620, for example, an inlet end of the chamber 620, corresponding to a first end portion of the piezoelectric diaphragm 610, regulating flow through the chamber 620 in a first direction. A second check valve 632 is positioned at a second side of the chamber 620, for example, an outlet end of the chamber 620, corresponding to a second end portion of the piezoelectric diaphragm 610, regulating flow through the chamber 620 in a second direction. Application of a voltage, or an increase in voltage, causes deformation of the piezo-ceramic disc 615 and a corresponding upstroke of the membrane 625 and diaphragm 635, as shown in FIG. 6B. This upstroke of the disc 615 corresponding to a supply stroke draws fluid into the chamber 620 through the first check valve 631 to fill the chamber 620. Release of the voltage, or a decrease in voltage, causes deformation of the disc 615 and a corresponding down stroke, as shown in FIG. 6C. This down stroke of the disc 615 corresponding to a pressure stroke displaces fluid out of, or expels fluid from the chamber 620 through the second check valve 632. This pumping cycle can be repeated to continue to pump fluid into and out of, or through, the chamber 620.

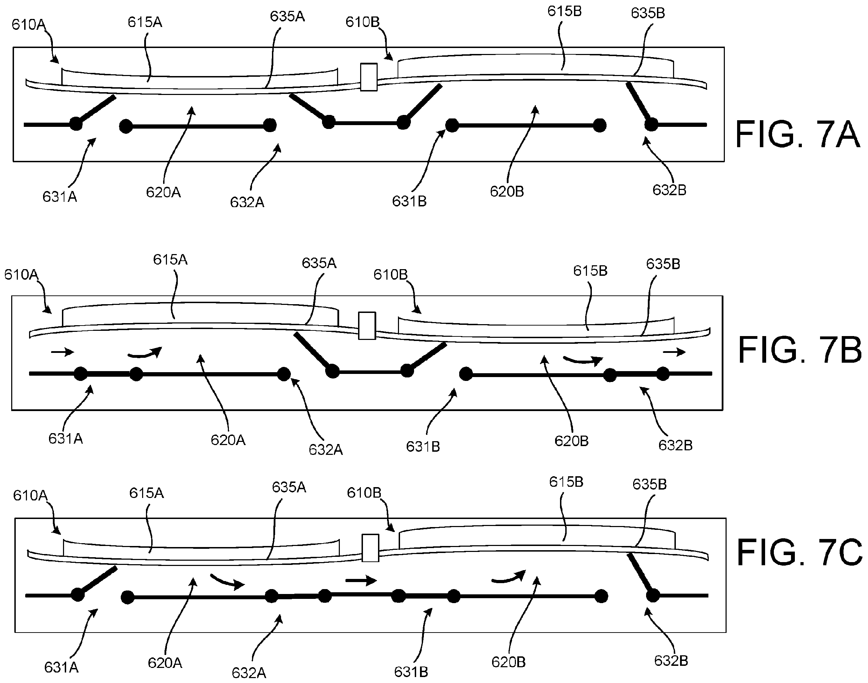

FIGS. 7A-7C schematically illustrate operation of a dual piezoelectric pump and valve manifold device. In particular, FIGS. 7A-7C illustrate operation of a dual piezoelectric pump and valve device through first, second and third phases of a pumping cycle of fluid through the dual piezoelectric pump and valve device.

In the first phase shown in FIG. 7A, a first check valve 631A and a second check valve 632A are in a closed position such that fluid does not flow into or out of a first chamber 620A corresponding to a first piezoelectric diaphragm 610A. Similarly, a first check valve 631B and a second check valve 632B are in a closed position such that fluid does not flow into or out of a second chamber 620B corresponding to a second piezoelectric diaphragm 610B.

In response to an application of voltage, a piezo-ceramic disc 615A and membrane 635A of the first piezoelectric diaphragm 610A perform an upstroke, or supply stroke, and a piezo-ceramic disc 615B and membrane 635B of the second piezoelectric diaphragm 610B perform a downstroke, or pressure stroke, from the respective first phase positions shown in FIG. 7A to the respective second phase positions shown in FIG. 7B. Voltage may be applied to the piezo-ceramic disc 615A based on, for example, a fluid pressure and/or a fluid flow rate measured by one of the pressure sensors included in the fluidic architecture described above. Upstroke of the first piezoelectric diaphragm 610A decreases a pressure in the first chamber 620A, opening the first check valve 631A and allowing fluid to flow through the first check valve 631A and into the first chamber 620A, while the second check valve 632A remains closed. Downstroke of the second piezoelectric diaphragm 610B increases a pressure in the second chamber 620B, opening the second check valve 632B and allowing fluid to flow out of the second chamber 620B and through the second check valve 632B, while the first check valve 631B remains closed.

In response to removal of the voltage, the piezo-ceramic disc 615A and membrane 635A of the first piezoelectric diaphragm 610A perform a downstroke, or pressure stroke, and the piezo-ceramic disc 615B and membrane 635B of the second piezoelectric diaphragm 610B perform an upstroke, or supply stroke, from the respective second phase positions shown in FIG. 7B to the respective third phase positions shown in FIG. 7C. Removal of the voltage applied to the piezo-ceramic disc 615A may be based on, for example, a fluid pressure and/or a fluid flow rate measured by one of the pressure sensors included in the fluidic architecture described above. Downstroke of the first piezoelectric diaphragm 610A increases a pressure in the first chamber 620A, closing the first check valve 631A and opening the second check valve 632A, allowing fluid to flow through the second check valve 632A and into the fluid channel toward the second chamber 620B. Upstroke of the second piezoelectric diaphragm 610B decreases a pressure in the second chamber 620B, opening the first check valve 631B and allowing fluid to flow into the second chamber 620B, while the second check valve 632B remains closed.

Thus, the first, second and third phases of the pumping cycle of the dual piezoelectric pump and valve device shown in FIGS. 7A-7C illustrate the refilling of fluid in the first chamber 620A and the discharge of fluid accumulated in the second chamber 620B in going from the first phase (FIG. 7A) to the second phase (FIG. 7B), and the discharge of fluid accumulated in the first chamber 620A and the refilling of fluid into the second chamber 620B in going from the second phase (FIG. 7B) to the third phase (FIG. 7C).

In the example described above with respect to FIGs. 7A-7C, the dual piezoelectric pump and valve device includes a first check valve 631A, 631B and a second check valve 632A, 632B respectively associated with the flow through each chamber 620A, 620B. In some implementations, operation of the second check valve 632A of the first chamber 620A and the first check valve 631B of the second chamber 620B can be replaced with a single valve (not shown in FIGs. 7A-7C) that can control the flow between the first chamber 620A and the second chamber 620B in a similar manner to that which is described above with respect to FIGs. 7A-7C.

In some examples, a current-mode sensing method may be applied to determine pressure in a piezoelectric diaphragm pump. As current and pressure are linearly interrelated, pressure can be inferred from the amount of current required to move the diaphragm. In this type of current-mode sensing, pressure can be sensed at each pumping cycle as described above, based on the amount of current required to move the diaphragm and fill/empty the respective chamber.

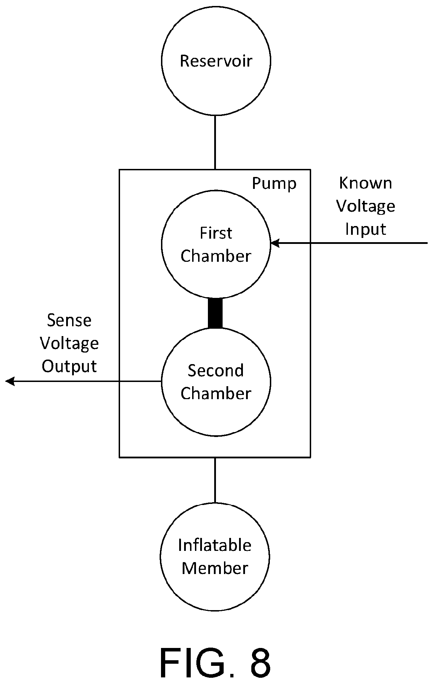

In some examples, an induced-response method may be applied to determine pressure. The induced-response method may make use of the ability of piezoelectric materials to convert movement into voltage (in addition to moving in response to the application of electrical stimulus, as described above). As the electromechanical actuation and responses of piezoelectric materials are associated with alternating current (AC) signals, the above-described use of the pump as a sensor (in, for example, the piezoelectric diaphragm pump as described above) can only measure changes in pressure. In some examples, this can be overcome by controlling an input to one fluid chamber, and measuring an output at another fluid chamber. FIG. 8 is a schematic diagram of an example dual piezoelectric pump manifold configuration, such as the example dual piezoelectric pump and valve device shown in FIGS. 7A-7C, having multiple chambers arranged in series. In this example arrangement, the first chamber (for example, the first chamber 620A) may be connected to the second chamber (for example, the second chamber 620B) by a fluid passageway. A known stimulus (i.e., a known voltage level, or a known pulse level) is input at the first chamber, and the output at the second chamber (a voltage level, or a pulse magnitude) is detected. In some examples, a static pressure can be determined based on a known pulse input applied to the first chamber, and the resultant pulse output measured at the second chamber.

As established above, the ability to accurately measure and monitor pressure in an implantable fluid operated device as described herein is essential for proper operation of the device and device efficacy, and to ensure patient safety. In some situations, it may be necessary to also be able to identify atmospheric pressure, and to adjust operation of the device accordingly to account for differences from a calibrated atmospheric pressure level in operation and control of the device. For example, the example devices 100 described above operate based on a principle of differential pressure. With a relatively high pressure in the reservoir 102, a relatively low pressure will be present in the inflatable member 104. Similarly, with a relatively low pressure in the reservoir 102, a relatively high pressure will be present in the inflatable member 104. If the device 100 is calibrated, for example, at sea level, variances in atmospheric pressure (i.e., above or below sea level) may affect pressure measurement and monitoring in the fluid channels of the device 100, and may affect operation of the device 100. Control of fluid pressure within the device 100, and in particular at various different positions within the device 100, may provide for monitoring of pressure within the device 100 and control of device operation independent of atmospheric pressure.

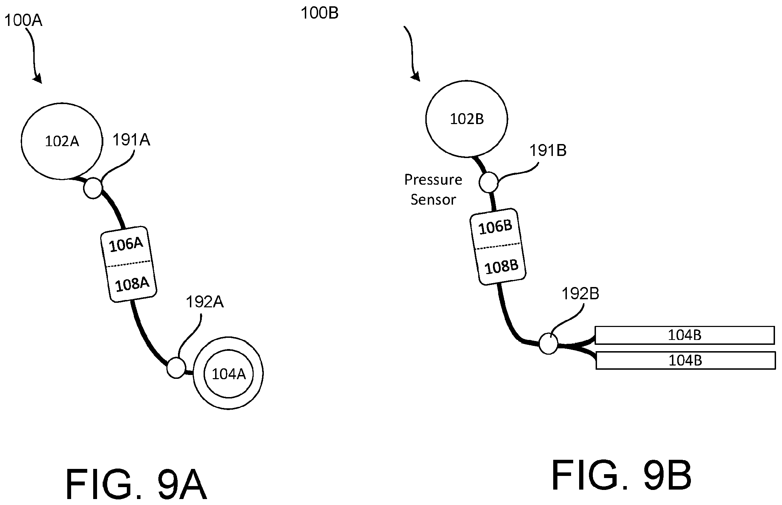

For example, absent a mechanism for accounting for atmospheric pressure changes, spikes, and the like, an increase or a decrease in atmospheric pressure (from the calibration pressure) may cause the device 100 to incorrectly pump fluid to the inflatable member 104, or back to the reservoir 102, to account for the offset in atmospheric pressure. FIGS. 9A and 9B illustrate the example devices 100 described above, in the form of the artificial urinary sphincter 100A and the example inflatable penile prosthesis 100B. Each of the example devices 100 includes inline pressure sensors. For example, a first inline pressure sensor 191 (191A, 191B) is positioned close to the reservoir 102, and a second inline pressure sensor 192 (192A, 192B) is positioned close to the inflatable member 104 of each device 100.

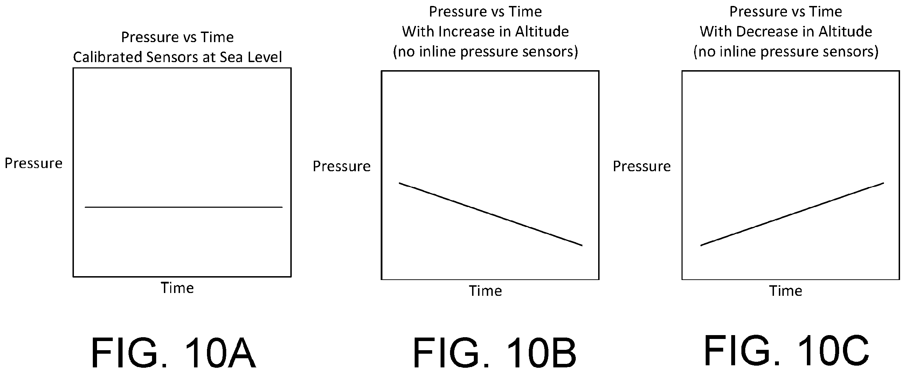

When calibrated, for example, at sea level, any pressure differential between the reservoir 102 and the inflatable member 104 is accounted for, or offset, or known, based on a pressure measurement provided by the first pressure sensor 191 and the second pressure sensor 192. When functioning properly, the first and second pressure sensors 191, 192 should experience the same decrease or increase in pressure in response to a sudden increase in altitude, or a sudden decrease in altitude, thus maintaining a substantially constant pressure level, as illustrated by the graph shown in FIG. 10A. The use of inline pressure sensors as described may allow for measurements taken by the first and second pressure sensors 191, 192 to be transmitted to the electronic control system 108, to be monitored, and in the event of an increase or decrease in pressure, internal algorithms (for example, applied or carried out by components of the control system 108 of the device 100) can use the pressure measurements to account for the difference and adapt the pumping of fluid through the device to maintain a proper inflated/deflated state of the inflatable member 104.

In particular, the graph shown in FIG. 10B illustrates that, in response to an increase in altitude, a decrease in system pressure is experienced. Without the first and second inline pressure sensors 191, 192 as described above, and a control algorithm that provides for correction of pressure levels to account for changes in altitude, the observed decrease in pressure could trigger the device 100 to (erroneously) increase pumping of fluid to the inflatable member 104. This may cause over-pressurization of the cuff 104A and damage to the urethra and/or device failure, or unintended inflation of the inflatable cylinders 104B. Similarly, the graph shown in FIG. 10C illustrates that, in response to a decrease in altitude, an increase in system pressure is experienced. Without the first and second inline pressure sensors 191, 192 as described above, and a control algorithm that provides for correction of pressure levels to account for changes in altitude, the observed increase in pressure could trigger the device 100 to (erroneously) decrease pumping of fluid to the inflatable member 104/deflate the inflatable member 104 and re-direct fluid from the inflatable member 104 back to the reservoir 102. This may result in an under-pressurization of the cuff 104A on the urethra and patient leakage, or unintended deflation of the inflatable cylinders 104B.

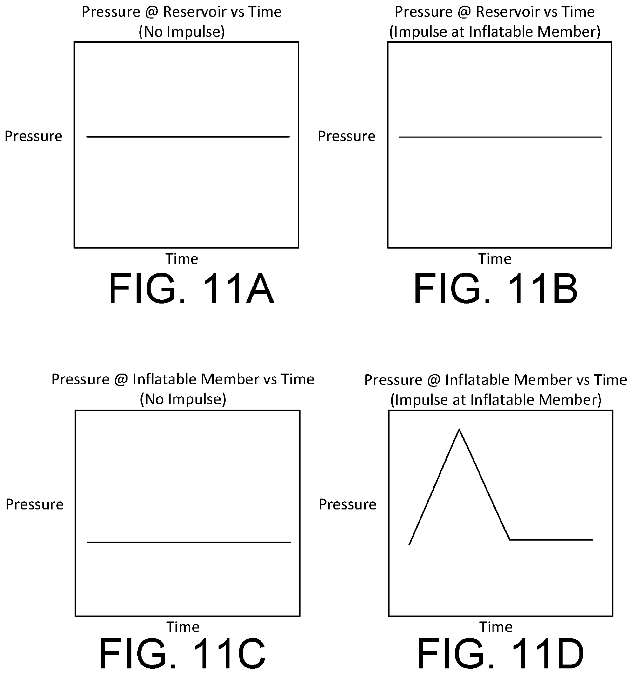

The graphs shown in FIGS. 11A-11D illustrate the effect of a single, abrupt impulse or impact experienced at the inflatable member 104 due to various physical actions such as, for example, exercise and the like which may temporarily impinge on the inflatable member 104 and cause an intermittent spike in pressure. Under normal, calibrated conditions (and in the absence of an impulse as described above), any pressure differential between the reservoir 102 and the inflatable member 104 is accounted for, or offset, or known, based on pressure measurements provided by the first and second pressure sensors 191, 192 as described above, and as shown in FIGS. 11A and 11C. Further, based on the inline placement of the first and second pressure sensors 191, 192, the system may detect that, in this scenario the sudden spike in pressure is detected only by the second pressure sensor 192 (at or near the inflatable member 104) as shown in FIG. 11D, but not by the first pressure sensor 191 (at or near the reservoir 102) as shown in FIG. 11B. The system may then take action based on an established decision algorithm to increase pumping action, decrease pumping action, or take no action. For example, if continued pressure monitoring detects that the pressure increase is not sustained over a period of time, and that pressure returns to within the expected calibrated range as shown in FIG. 11D, no action is taken. This may allow the device 100 to adapt to specific use scenarios relatively quickly, while also enhancing patient safety and comfort.

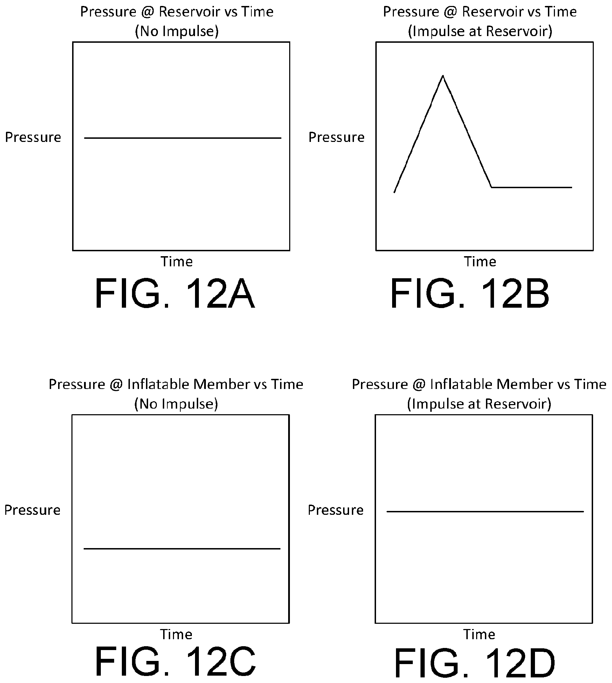

The graphs shown in FIGS. 12A-12D illustrate the effect of a single, abrupt impulse or impact experienced at the reservoir 102 due to various physical actions such as, for example, a fall and the like which may temporarily impinge on the reservoir 102 and cause an intermittent spike in pressure. Under normal, calibrated conditions (and in the absence of an impulse as described above), any pressure differential between the reservoir 102 and the inflatable member 104 is accounted for, or offset, or known, based on pressure measurements provided by the first and second pressure sensors 191, 192 as described above, and as shown in FIGS. 12A and 12C. Further, based on the inline placement of the first and second pressure sensors 191, 192, the system may detect that, in this scenario the sudden spike in pressure is detected only by the first pressure sensor 191 (at or near the reservoir 102) as shown in FIG. 12B, but not by the second pressure sensor 192 (at or near the inflatable member 104) as shown in FIG. 12D. The system may then take action based on an established decision algorithm to increase pumping action, decrease pumping action, or take no action. For example, if continued pressure monitoring detects that the pressure increase is not sustained over a period of time, and that pressure returns to within the expected calibrated range as shown in FIG. 12B, no action is taken. This may allow the device 100 to adapt to specific use scenarios relatively quickly, while also enhancing patient safety and comfort.

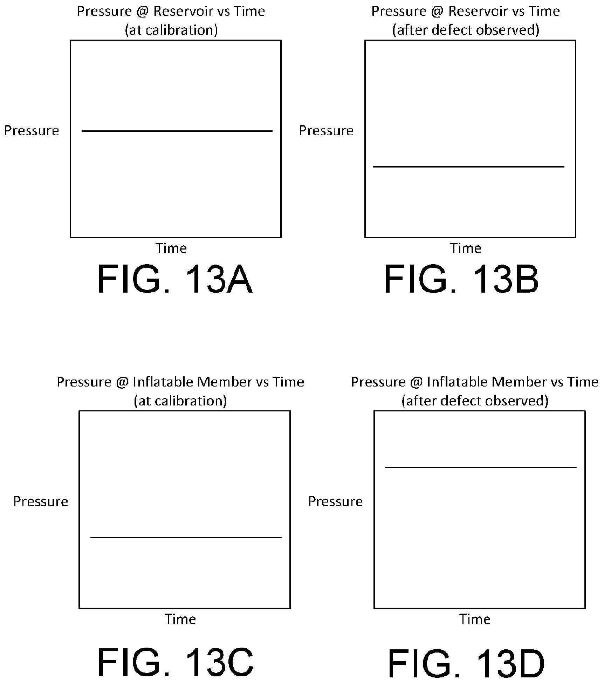

The graphs shown in FIGS. 13A-13D illustrate the effect of a relatively long term different or drift in set pressure values between the reservoir 102 and the inflatable member 104, or a time to reach the set pressure values is noticeably increased. These events may be indicative of a blockage in one of the fluid passageways of the device 100, or other type of damage or malfunction of the device 100, and may provide notification to the patient and/or physician for correction. In normal operation, an offset between pressure levels measured by the first and second inline pressure sensors 191, 192 should remain essentially constant, as shown in FIGS. 13A and 13C. A component failure, a leak, a blockage or other such disruption would generate a surge in pressure, or a decrease in pressure, based on the type of failure and the location of the failure within the device 100, as shown in FIGS. 13B and 13D. The detection of a sustained decrease or surge in pressure can provide an alert to the patient and/or to the physician to provide for correction, thus enhancing patient safety and comfort.

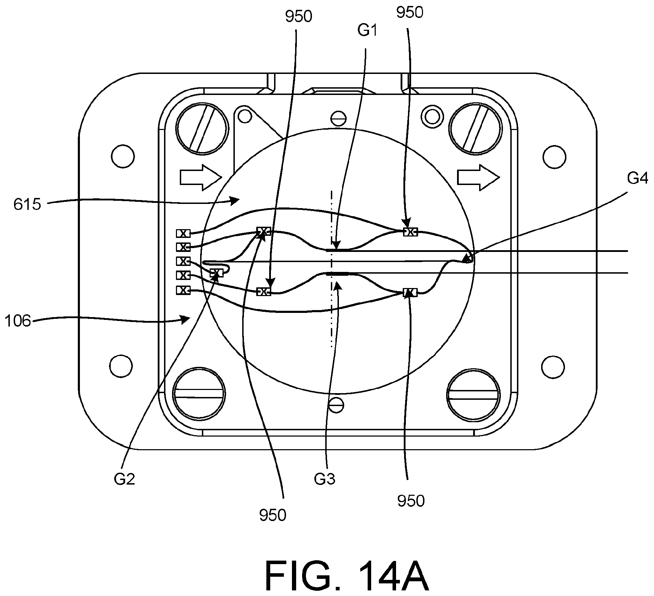

As noted above, the example inline pressure sensors 191, 192 shown in FIGS. 9A and 9B may be positioned in the fluid passageways of the implantable fluid activated device 100. In some examples, the inline pressure sensors 191, 192 can include a diaphragm positioned in the fluid passageway. For example, the first pressure sensor 191 can include a diaphragm positioned in the fluid passageway and facing the reservoir 102, and the second pressure sensor 192 can include a diaphragm positioned within the fluid passageway and facing the inflatable member 104. Deflection of the diaphragm can be detected/measured and an algorithm (for example carried out by the electronic control system 108) can covert the detected movement or deflection of the diaphragm into a pressure. In some examples, the deflection of the diaphragm may be measured by a strain gauge positioned on the diaphragm. FIG. 14A illustrates one example of strain gauges 950 mounted on the diaphragm within a fluid passageway of the pump assembly 106. In some examples, the diaphragm is made of a bio-compatible material such as, for example, Titanium. In some examples, the diaphragm is coated in an elastic material such as, for example, a silicone material, a ceramic material and the like, that provides a moisture barrier on the diaphragm while also allowing for the transfer of a signal from the strain gauge. In some examples, the device 100 can communicate with an external device (for example, through a communication module of the electronic control system 108). Communication with the external device can provide for the exchange of information such as, for example, atmospheric pressure readings (that allow the internal device 100 to adjust pressures as necessary), internal pressure measurements, alerts and the like.

As described above, the ability to detect other than normal pressure level(s) in the device 100, and to adapt the operation of the device 100 in response to detection of the other than normal pressure level(s) enhances patient safety and device efficacy. For example, as described above with respect to FIGS. 11A-11D, a detected spike or increase in pressure may cause the device 100 to adjust pumping action. In some situations, the decision to adjust pumping action may be based on an observed duration of the increased pressure. For example, in the case of the artificial urinary sphincter 100A, the insertion of a catheter can cause a relatively rapid increase in pressure, particularly if the cuff 104A has not been deflated prior to insertion of the catheter. For example, in some situations, the patient may be incapacitated and/or unable to communicate the presence of the implanted artificial urinary sphincter. Insertion of the catheter with the cuff 104A in the inflated condition causes a rapid buildup of pressure in the device 100A, that is sustained and/or continues to increase as the catheter is inserted. In this example, the detection of this type of sustained pressure spike may cause the electronic control system 108A to actuate the pump assembly 106A to deflate the cuff 104A, thus opening the cuff 104A and allowing the catheter to be inserted without damaging the cuff 104A and/or the urethra.

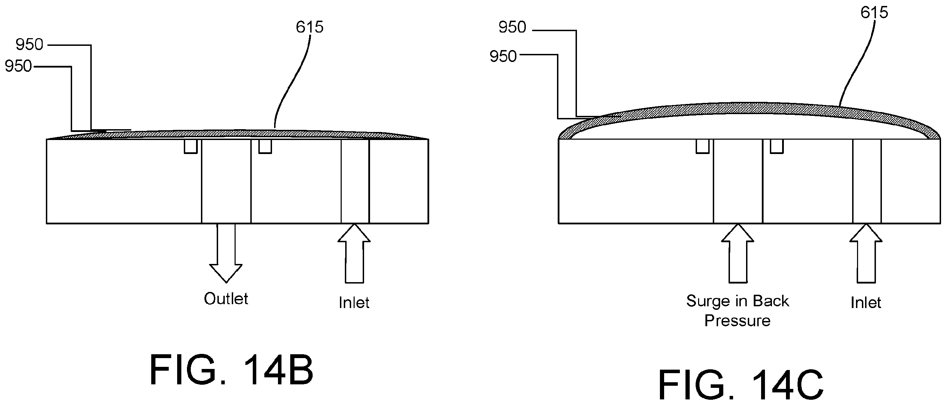

In some examples, the spike in pressure is detected by a pressure sensor within the fluid passageways of the device, including, for example, a piezoelectric element as described above, a pressure transducer, and the like. In some examples, the spike in pressure is detected based on dynamic pressure changes in a piezoelectric element. As described above, diaphragms placed positioned in the fluid passageway facing the reservoir 102A and facing the cuff 104A are deflected as fluid pressure changes. A normal state and a deflected state of the example diaphragm 615 is shown in FIGS. 14B and 14C. The dynamic pressure in response to insertion of a catheter as described above generates a voltage change that is measurable by the strain gauge(s) 950. The voltage change is indicative of a change in pressure caused by the insertion of the catheter. The electronic control system 108 can process the detected change in pressure and control the pump assembly 106 to provide for deflation/opening of the cuff 104A.

## Claims

1. An implantable fluid operated inflatable device (100), comprising:
a fluid reservoir (102);
an inflatable member (104);
a pump assembly (106) configured to transfer fluid between the fluid reservoir (102) and the inflatable member (104), including:
a manifold (400), including:
a housing (410);
at least one valve and at least one pump positioned in a fluid passageway formed in the housing;
a first fluid port (430A) in fluidic communication with the fluid reservoir (102); and
a second fluid port (430B) in fluidic communication with the inflatable member (104);
an electronic control system (550) configured to control operation of the pump assembly (106); and
at least one pressure sensing device configured to communicate with the electronic control system (550),
wherein the at least one valve and the at least one pump includes:
a first pump (450A) and a first valve (460A) positioned in a first fluid passageway (490A) and in fluidic communication with the first fluid port (430A); and
a second pump (450B) and a second valve (460B) positioned in a second fluid passageway (490B) and in fluidic communication with the second fluid port (430B);
wherein the first pump (450A) and the first valve (460A) are configured to direct fluid out of the manifold (400) through the first fluid port (430A) to the reservoir (102) of the fluid operated device (100), and the second pump (450B) and the second valve (460B) are configured to direct fluid out of the manifold (400) through the second fluid port (430B) to the inflatable member (104) of the fluid operated device (100).

2. The implantable fluid operated device (100) of claim 1, wherein the at least one pressure sensing device includes:
a first pressure sensing device (420A) positioned in the first fluid passageway (490A) and configured to measure a pressure of fluid flowing through the first fluid port (430A) and to transmit the measured pressure to the electronic control system (550A); and
a second pressure sensing device (420B) positioned in the second fluid passageway (490B) and configured to measure a pressure of fluid flowing through the second fluid port (430B) and to transmit the measured pressure to the electronic control system.

3. The implantable fluid operated inflatable device (100) of claim 1 or 2, wherein the at least one valve and the at least one pump includes a dual piezoelectric pump, including:
a first piezoelectric pump;
a second piezoelectric pump; and
a fluid channel providing for fluidic communication between the first piezoelectric pump and the second piezoelectric pump.

4. The implantable fluid operated inflatable device (100) of claim 3, wherein
the first piezoelectric pump includes:
a first chamber (620A);
a first piezoelectric diaphragm (610A) positioned along an edge portion of the first chamber (620A) and configured to have a voltage selectively applied thereto in response to a fluid pressure detected by at least one of the first pressure sensing device (420A) or the second pressure sensing device (420B);
a first check valve (631A) at an inlet end of the first chamber (620A); and
a second check valve (632A) at an outlet end of the first chamber (620A), the second check valve (632A) of the first piezoelectric pump selectively providing fluidic communication between the first chamber (620A) and the fluid channel; and
the second piezoelectric pump includes:
a second chamber (620B);
a second piezoelectric diaphragm (610B) positioned along an edge portion of the second chamber (620B) and configured to have a voltage selectively applied thereto in response to a fluid pressure detected by at least one of the first pressure sensing device (420A) or the second pressure sensing device (420B);
a first check valve (631B) at an inlet end of the second chamber, the first check valve (631B) of the second piezoelectric pump selectively providing fluidic communication between the fluid channel and the second chamber (620B); and
a second check valve (632B) at an outlet end of the second chamber (620B).

5. The implantable fluid operated inflatable device (100) of claim 4, wherein a pumping cycle of the dual piezoelectric pump includes:
a first phase including a supply stroke of the first piezoelectric diaphragm (610A) in coordination with a pressure stroke of the second piezoelectric diaphragm (610B); and
a second phase including a pressure stroke of the first piezoelectric diaphragm (610A) in coordination with a supply stroke of the second piezoelectric diaphragm (610B).

6. The implantable fluid operated inflatable device (100) of claim 5, wherein
in the first phase, fluid is drawn into the first chamber (620A) through the first check valve (631A) of the first piezoelectric pump, and fluid is expelled from the second chamber (620B) through the second check valve (632B) of the second piezoelectric pump; and
in the second phase, fluid is expelled from the first chamber (620A) and into the fluid channel through the second check valve (632A) of the first piezoelectric pump, and fluid is drawn from the fluid channel into the second chamber (620B) through the first check valve (631B) of the second piezoelectric pump.

7. The implantable fluid operated inflatable device (100) of any of claims 1 to 6, wherein the housing (410) of the manifold (400) is made of an injection molded metal material, with the at least one pump and the at least one valve positioned in a sealed fluid passageway defined in the injection molded metal material, such that the manifold (400) is a hermetic manifold.

8. The implantable fluid operated inflatable device (100) of any of claims 1 to 6, wherein the pump assembly (106) includes a pump assembly housing, and wherein the manifold (400) and the electronic control system are received in the pump assembly housing.

9. The implantable fluid operated inflatable device (100) of claim 8, wherein the manifold (400) is a hermetic manifold, such that components of the electronic control system (550) within the pump assembly housing are isolated from fluid flowing through the hermetic manifold.

10. The implantable fluid operated inflatable device (100) of claim 1, wherein the at least one pressure sensing device includes:
a first pressure sensing device (420A) positioned proximate a fluid port of the reservoir (102); and
a second pressure sensing device (420B) positioned proximate a fluid port of the inflatable member (104).

11. The implantable fluid operated inflatable device (100) of claim 10, wherein
the first pressure sensing device (420A) includes:
a first diaphragm positioned in a fluid passageway proximate the reservoir (102), facing the reservoir (102); and
at least one first strain gauge (950) mounted on the first diaphragm (615), the at least one first strain gauge (950) being configured to measure a deflection of the first diaphragm (615) and to transmit the measured deflection to the electronic control system (550); and
the second pressure sensing device includes:
a second diaphragm positioned in a fluid passageway proximate the fluid port of the inflatable member (104), facing the inflatable member (104); and
at least one second strain gauge mounted on the second diaphragm, the at least one second strain gauge being configured to measure a deflection of the second diaphragm and to transmit the measured deflection to the electronic control system.

12. The implantable fluid operated inflatable device (100) of any of claims 1 to 10, wherein the at least one sensing device includes at least one piezoelectric element positioned in a fluid passageway of the implantable fluid operated device (100) and configured to sense a fluid pressure level in the fluid passageway based on an input voltage level applied to the piezoelectric element and an output voltage level measured at the piezoelectric element.

13. The implantable fluid operated device (100) of any of claims 1 to 12, wherein the electronic control system (550) includes a printed circuit board (520) including a processor configured to:
receive pressure level measurements from the at least one sensing device;
apply a control algorithm based on the received pressure level measurements; and
control operation of the at least one valve and the at least one pump in accordance with the applied control algorithm.

14. The implantable fluid operated inflatable device (100) of any of claims 1 to 13, wherein the implantable fluid operated device (100) is an artificial urinary sphincter (100A) or an inflatable penile prosthesis (100B).

## Patentansprüche

1. Implantierbare fluidbetriebene aufblasbare Vorrichtung (100), umfassend:
einen Fluidreservoir (102);
ein aufblasbares Element (104);
eine Pumpenbaugruppe (106), die dazu konfiguriert ist, Fluid zwischen dem Fluidreservoir (102) und dem aufblasbaren Element (104) zu transferieren, umfassend:
einen Verteiler (400), umfassend:
ein Gehäuse (410);
mindestens ein Ventil und mindestens eine Pumpe, die in einem Fluiddurchgang positioniert sind, der in dem Gehäuse gebildet ist;
einen ersten Fluidanschluss (430A) in Fluidverbindung mit dem Fluidreservoir (102); und
einen zweiten Fluidanschluss (430B) in Fluidverbindung mit dem aufblasbaren Element (104);
ein elektronisches Steuersystem (550), das dazu konfiguriert ist, den Betrieb der Pumpenbaugruppe (106) zu steuern; und
mindestens eine Druckerfassungsvorrichtung, die dazu konfiguriert ist, mit dem elektronischen Steuersystem (550) zu kommunizieren,
wobei das mindestens eine Ventil und die mindestens eine Pumpe Folgendes beinhalten:
eine erste Pumpe (450A) und ein erstes Ventil (460A), die in einem ersten Fluiddurchgang (490A) positioniert sind und mit dem ersten Fluidanschluss (430A) in Fluidverbindung stehen; und
eine zweite Pumpe (450B) und ein zweites Ventil (460B), die in einem zweiten Fluiddurchgang (490B) positioniert sind und mit dem zweiten Fluidanschluss (430B) in Fluidverbindung stehen;
wobei die erste Pumpe (450A) und das erste Ventil (460A) dazu konfiguriert sind, Fluid aus dem Verteiler (400) durch den ersten Fluidanschluss (430A) zu dem Reservoir (102) der fluidbetriebenen Vorrichtung (100) zu leiten, und die zweite Pumpe (450B) und das zweite Ventil (460B) dazu konfiguriert sind, Fluid aus dem Verteiler (400) durch den zweiten Fluidanschluss (430B) zu dem aufblasbaren Element (104) der fluidbetriebenen Vorrichtung (100) zu leiten.

2. Implantierbare fluidbetriebene Vorrichtung (100) nach Anspruch 1, wobei die mindestens eine Druckerfassungsvorrichtung Folgendes beinhaltet:
eine erste Druckerfassungsvorrichtung (420A), die in dem ersten Fluiddurchgang (490A) positioniert und dazu konfiguriert ist, einen Druck von Fluid, das durch den ersten Fluidanschluss (430A) strömt, zu messen und den gemessenen Druck an das elektronische Steuersystem (550A) zu übertragen; und
eine zweite Druckerfassungsvorrichtung (420B), die in dem zweiten Fluiddurchgang (490B) positioniert und dazu konfiguriert ist, einen Druck von Fluid, das durch den zweiten Fluidanschluss (430B) strömt, zu messen und den gemessenen Druck an das elektronische Steuersystem zu übertragen.

3. Implantierbare fluidbetriebene aufblasbare Vorrichtung (100) nach Anspruch 1 oder 2, wobei das mindestens eine Ventil und die mindestens eine Pumpe eine piezoelektrische Dual-Pumpe beinhalten, die Folgendes beinhaltet:
eine erste piezoelektrische Pumpe;
eine zweite piezoelektrische Pumpe; und
einen Fluidkanal, der eine Fluidverbindung zwischen der ersten piezoelektrischen Pumpe und der zweiten piezoelektrischen Pumpe bereitstellt.

4. Implantierbare fluidbetriebene aufblasbare Vorrichtung (100) nach Anspruch 3, wobei
die erste piezoelektrische Pumpe Folgendes beinhaltet:
eine erste Kammer (620A);
ein erstes piezoelektrisches Diaphragma (610A), die entlang eines Kantenabschnitts der ersten Kammer (620A) positioniert und dazu konfiguriert ist, eine Spannung aufzuweisen, die als Reaktion auf einen Fluiddruck, der von mindestens einer von der ersten Druckerfassungsvorrichtung (420A) oder der zweiten Druckerfassungsvorrichtung (420B) detektiert wird, selektiv daran angelegt wird;
ein erstes Rückschlagventil (631A) an einem Einlassende der ersten Kammer (620A); und
ein zweites Rückschlagventil (632A) an einem Auslassende der ersten Kammer (620A), wobei das zweite Rückschlagventil (632A) der ersten piezoelektrischen Pumpe selektiv eine Fluidverbindung zwischen der ersten Kammer (620A) und dem Fluidkanal bereitstellt; und
die zweite piezoelektrische Pumpe Folgendes beinhaltet:
eine zweite Kammer (620B);
ein zweites piezoelektrischea Diaphragma (610B), die entlang eines Kantenabschnitts der zweiten Kammer (620B) positioniert und dazu konfiguriert ist, eine Spannung aufzuweisen, die als Reaktion auf einen Fluiddruck, der von mindestens einer von der ersten Druckerfassungsvorrichtung (420A) oder der zweiten Druckerfassungsvorrichtung (420B) detektiert wird, selektiv daran angelegt wird;
ein erstes Rückschlagventil (631B) an einem Einlassende der zweiten Kammer, wobei das erste Rückschlagventil (631B) der zweiten piezoelektrischen Pumpe selektiv eine Fluidverbindung zwischen dem Fluidkanal und der zweiten Kammer (620B) bereitstellt; und
ein zweites Rückschlagventil (632B) an einem Auslassende der zweiten Kammer (620B).

5. Implantierbare fluidbetriebene aufblasbare Vorrichtung (100) nach Anspruch 4, wobei ein Pumpzyklus der piezoelektrischen Dual-Pumpe Folgendes beinhaltet:
eine erste Phase, die einen Einspeisungshub des ersten piezoelektrischen Diaphragmas (610A) in Übereinstimmung mit einem Druckhub des zweiten piezoelektrischen Diaphragmas (610B) beinhaltet; und
eine zweite Phase, die einen Druckhub des ersten piezoelektrischen Diaphragmas (610A) in Übereinstimmung mit einem Einspeisungshub des zweiten piezoelektrischen Diaphragmas (610B) beinhaltet.

6. Implantierbare fluidbetriebene aufblasbare Vorrichtung (100) nach Anspruch 5, wobei
in der ersten Phase Fluid durch das erste Rückschlagventil (631A) der ersten piezoelektrischen Pumpe in die erste Kammer (620A) gezogen wird und Fluid durch das zweite Rückschlagventil (632B) der zweiten piezoelektrischen Pumpe aus der zweiten Kammer (620B) ausgestoßen wird; und
in der zweiten Phase Fluid durch das zweite Rückschlagventil (632A) der ersten piezoelektrischen Pumpe aus der ersten Kammer (620A) und in den Fluidkanal ausgestoßen wird und Fluid durch das erste Rückschlagventil (631B) der zweiten piezoelektrischen Pumpe aus dem Fluidkanal in die zweite Kammer (620B) gezogen wird.

7. Implantierbare fluidbetriebene aufblasbare Vorrichtung (100) nach einem der Ansprüche 1 bis 6, wobei das Gehäuse (410) des Verteilers (400) aus einem spritzgegossenen Metallmaterial besteht, wobei die mindestens eine Pumpe und das mindestens eine Ventil in einem abgedichteten Fluiddurchgang positioniert sind, der in dem spritzgegossenen Metallmaterial definiert ist, sodass der Verteiler (400) ein hermetischer Verteiler ist.

8. Implantierbare fluidbetriebene aufblasbare Vorrichtung (100) nach einem der Ansprüche 1 bis 6, wobei die Pumpenbaugruppe (106) ein Pumpenbaugruppengehäuse beinhaltet und wobei der Verteiler (400) und das elektronische Steuersystem in dem Pumpenbaugruppengehäuse aufgenommen sind.

9. Implantierbare fluidbetriebene aufblasbare Vorrichtung (100) nach Anspruch 8, wobei der Verteiler (400) ein hermetischer Verteiler ist, sodass Komponenten des elektronischen Steuersystems (550) innerhalb des Pumpenbaugruppengehäuses von dem Fluid, das durch den hermetischen Verteiler strömt, isoliert sind.

10. Implantierbare fluidbetriebene aufblasbare Vorrichtung (100) nach Anspruch 1, wobei die mindestens eine Druckerfassungsvorrichtung Folgendes beinhaltet:
eine erste Druckerfassungsvorrichtung (420A), die in der Nähe eines Fluidanschlusses des Reservoirs (102) positioniert ist; und
eine zweite Druckerfassungsvorrichtung (420B), die in der Nähe eines Fluidanschlusses des aufblasbaren Elements (104) positioniert ist.

11. Implantierbare fluidbetriebene aufblasbare Vorrichtung (100) nach Anspruch 10, wobei
die erste Druckerfassungsvorrichtung (420A) Folgendes beinhaltet:
ein erstes Diaphragma, das in einem Fluiddurchgang in der Nähe des Reservoirs (102) positioniert und dem Reservoir (102) zugewandt ist; und
mindestens einen ersten Dehnungsmesser (950), der an dem ersten Diaphragma (615) montiert ist, wobei der mindestens eine erste Dehnungsmesser (950) dazu konfiguriert ist, eine Ablenkung des ersten Diaphragmas (615) zu messen und die gemessene Ablenkung an das elektronische Steuersystem (550) zu übertragen; und
die zweite Druckerfassungsvorrichtung Folgendes beinhaltet:
ein zweites Diaphragma, das in einem Fluiddurchgang in der Nähe des Fluidanschlusses des aufblasbaren Elements (104) positioniert und dem aufblasbaren Element (104) zugewandt ist; und
mindestens einen zweiten Dehnungsmesser, der an dem zweiten Diaphragma montiert ist, wobei der mindestens eine zweite Dehnungsmesser dazu konfiguriert ist, eine Ablenkung des zweiten Diaphragmas zu messen und die gemessene Ablenkung an das elektronische Steuersystem zu übertragen.

12. Implantierbare fluidbetriebene aufblasbare Vorrichtung (100) nach einem der Ansprüche 1 bis 10, wobei die mindestens eine Erfassungsvorrichtung mindestens ein piezoelektrisches Element beinhaltet, das in einem Fluiddurchgang der implantierbaren fluidbetriebenen Vorrichtung (100) positioniert und dazu konfiguriert ist, einen Fluiddruckpegel in dem Fluiddurchgang auf Grundlage eines Eingangsspannungspegels, der an das piezoelektrische Element angelegt wird, und eines Ausgangsspannungspegels, der an dem piezoelektrischen Element gemessen wird, zu erfassen.

13. Implantierbare fluidbetriebene Vorrichtung (100) nach einem der Ansprüche 1 bis 12, wobei das elektronische Steuersystem (550) eine Leiterplatte (520) beinhaltet, die einen Prozessor beinhaltet, der zu Folgendem konfiguriert ist:
Empfangen von Druckpegelmessungen von der mindestens einen Erfassungsvorrichtung;
Anwenden eines Steueralgorithmus auf Grundlage der empfangenen Druckpegelmessungen; und
Steuern des Betriebs des mindestens einen Ventils und der mindestens einen Pumpe gemäß dem angewendeten Steueralgorithmus.

14. Implantierbare fluidbetriebene aufblasbare Vorrichtung (100) nach einem der Ansprüche 1 bis 13, wobei die implantierbare fluidbetriebene Vorrichtung (100) ein artifizieller Sphinkter (100A) oder eine aufblasbare Penisprothese (100B) ist.

## Revendications

1. Dispositif gonflable actionné par fluide implantable (100), comprenant :
un réservoir de fluide (102) ;
un élément gonflable (104) ;
une assemblé de pompe (106) configuré pour transférer du fluide entre le réservoir de fluide (102) et l'élément gonflable (104), incluant :
un collecteur (400), incluant :
un logement (410) ;
au moins une soupape et au moins une pompe positionné dans une voie de passage de fluide formée dans le logement ;
un premier orifice de fluide (430A) en communication fluidique avec le réservoir de fluide (102) ; et
un second orifice de fluide (430B) en communication fluidique avec l'élément gonflable (104) ;
un système de commande électronique (550) configuré pour commander le fonctionnement de l'assembléensemble de pompe (106) ; et
au moins un dispositif de détection de pression configuré pour communiquer avec le système de commande électronique (550),
dans lequel l'au moins une soupape et l'au moins une pompe inclut :
une première pompe (450A) et une première soupape (460A) positionnées dans une première voie de passage de fluide (490A) et en communication fluidique avec le premier orifice de fluide (430A) ; et
une seconde pompe (450B) et une seconde soupape (460B) positionnées dans une seconde voie de passage de fluide (490B) et en communication fluidique avec le second orifice de fluide (430B) ; et
dans lequel a première pompe (450A) et la première soupape (460A) sont configurées pour diriger le fluide en sortie du collecteur (400) au travers du premier orifice de fluide (430A) jusqu'au réservoir (102) du dispositif gonflable actionné par fluide implantable (100), et la seconde pompe (450B) et la seconde soupape (460B) sont configurées pour diriger le fluide en sortie du collecteur (400) au travers du second orifice de fluide (430B) jusqu'à l'élément gonflable (104) du dispositif gonflable actionné par fluide implantable (100).

2. Dispositif gonflable actionné par fluide implantable (100) selon la revendication 1, dans lequel l'au moins un dispositif de détection de pression inclut :
un premier dispositif de détection de pression (420A) positionné dans la première voie de passage de fluide (490A) et configuré pour mesurer une pression d'un fluide qui s'écoule au travers du premier orifice de fluide (430A) et pour transmettre la pression mesurée au système de commande électronique (550A) ; et
un second dispositif de détection de pression (420B) positionné dans la seconde voie de passage de fluide (490B) et configuré pour mesurer une pression d'un fluide qui s'écoule au travers du second orifice de fluide (430B) et pour transmettre la pression mesurée au système de commande électronique.

3. Dispositif gonflable actionné par fluide implantable (100) selon la revendication 1 ou 2, dans lequel l'au moins une soupape et l'au moins une pompe inclut une pompe piézoélectrique double, incluant :
une première pompe piézoélectrique ;
une seconde pompe piézoélectrique ; et
un canal de fluide qui assure une communication fluidique entre la première pompe piézoélectrique et la seconde pompe piézoélectrique.

4. Dispositif gonflable actionné par fluide implantable (100) selon la revendication 3, dans lequel :
la première pompe piézoélectrique inclut :
une première chambre (620A) ;
un premier diaphragme piézoélectrique (610A) positionné le long d'une partie de bord de la première chambre (620A) et configuré de telle sorte qu'une tension lui soit appliquée de façon sélective en réponse à une pression de fluide détectée par au moins un dispositif de détection de pression parmi le premier dispositif de détection de pression (420A) et le second dispositif de détection de pression (420B) ;
un premier clapet antiretour (631A) au niveau d'une extrémité d'entrée de la première chambre (620A) ; et
un second clapet antiretour (632A) au niveau d'une extrémité de sortie de la première chambre (620A), le second clapet antiretour (632A) de la première pompe piézoélectrique assurant de façon sélective une communication fluidique entre la première chambre (620A) et le canal de fluide ; et
la seconde pompe piézoélectrique inclut :
une seconde chambre (620B) ;
un second diaphragme piézoélectrique (610B) positionné le long d'une partie de bord de la seconde chambre (620B) et configuré de telle sorte qu'une tension lui soit appliquée de façon sélective en réponse à une pression de fluide détectée par au moins un dispositif de détection de pression parmi le premier dispositif de détection de pression (420A) et le second dispositif de détection de pression (420B) ;
un premier clapet antiretour (631B) au niveau d'une extrémité d'entrée de la seconde chambre, le premier clapet antiretour (631B) de la seconde pompe piézoélectrique assurant de façon sélective une communication fluidique entre le canal de fluide et la seconde chambre (620B) ; et
un second clapet antiretour (632B) au niveau d'une extrémité de sortie de la seconde chambre (620B).

5. Dispositif gonflable actionné par fluide implantable (100) selon la revendication 4, dans lequel un cycle de pompage de la pompe piézoélectrique double inclut :
une première phase qui inclut une course d'alimentation du premier diaphragme piézoélectrique (610A) en coordination avec une course de mise sous pression du second diaphragme piézoélectrique (610B) ; et
une seconde phase qui inclut une course de mise sous pression du premier diaphragme piézoélectrique (610A) en coordination avec une course d'alimentation du second diaphragme piézoélectrique (610B).

6. Dispositif gonflable actionné par fluide implantable (100) selon la revendication 5, dans lequel :
dans la première phase, du fluide est entraîné à l'intérieur de la première chambre (620A) au travers du premier clapet antiretour (631A) de la première pompe piézoélectrique et du fluide est expulsé depuis la seconde chambre (620B) au travers du second clapet antiretour (632B) de la seconde pompe piézoélectrique ; et
dans la seconde phase, du fluide est expulsé depuis la première chambre (620A) et à l'intérieur du canal de fluide au travers du second clapet antiretour (632A) de la première pompe piézoélectrique et du fluide est entraîné depuis le canal de fluide à l'intérieur de la seconde chambre (620B) au travers du premier clapet antiretour (631B) de la seconde pompe piézoélectrique.

7. Dispositif gonflable actionné par fluide implantable (100) selon l'une quelconque des revendications 1 à 6, dans lequel le logement (410) du collecteur (400) est réalisé en un matériau métallique moulé par injection, l'au moins une pompe et l'au moins une soupape étant positionnés dans une voie de passage de fluide étanche qui est définie dans le matériau métallique moulé par injection de telle sorte que le collecteur (400) soit un collecteur hermétique.

8. Dispositif gonflable actionné par fluide implantable (100) selon l'une quelconque des revendications 1 à 6, dans lequel l'ensemble de pompes (106) inclut un carter d'ensemble de pompes et dans lequel le collecteur (400) et le système de commande électronique sont reçus dans le carter d'ensemble de pompes.

9. Dispositif gonflable actionné par fluide implantable (100) selon la revendication 8, dans lequel le collecteur (400) est un collecteur hermétique de telle sorte que des composants du système de commande électronique (550) à l'intérieur du carter d'ensemble de pompes soient isolés du fluide qui s'écoule au travers du collecteur hermétique.

10. Dispositif gonflable actionné par fluide implantable (100) selon la revendication 1, dans lequel l'au moins un dispositif de détection de pression inclut :
un premier dispositif de détection de pression (420A) positionné à proximité d'un orifice de fluide du réservoir (102) ; et
un second dispositif de détection de pression (420B) positionné à proximité d'un orifice de fluide de l'élément gonflable (104).

11. Dispositif gonflable actionné par fluide implantable (100) selon la revendication 10, dans lequel :
le premier dispositif de détection de pression (420A) inclut :
un premier diaphragme positionné dans une voie de passage de fluide à proximité du réservoir (102), faisant face au réservoir (102) ; et
au moins une première jauge de contraintes (950) montée sur le premier diaphragme (615), l'au moins une première jauge de contraintes (950) étant configurée pour mesurer une déviation du premier diaphragme (615) et pour transmettre la déviation mesurée au système de commande électronique (550) ; et
le second dispositif de détection de pression inclut :
un second diaphragme positionné dans une voie de passage de fluide à proximité de l'orifice de fluide de l'élément gonflable (104), faisant face à l'élément gonflable (104) ; et
au moins une seconde jauge de contraintes montée sur le second diaphragme, l'au moins une seconde jauge de contraintes étant configurée pour mesurer une déviation du second diaphragme et pour transmettre la déviation mesurée au système de commande électronique.

12. Dispositif gonflable actionné par fluide implantable (100) selon l'une quelconque des revendications 1 à 10, dans lequel l'au moins un dispositif de détection de pression inclut au moins un élément piézoélectrique positionné dans une voie de passage de fluide du dispositif gonflable actionné par fluide implantable (100) et configuré pour détecter un niveau de pression de fluide dans la voie de passage de fluide sur la base d'un niveau de tension d'entrée qui est appliqué sur l'élément piézoélectrique et d'un niveau de tension de sortie qui est mesuré au niveau de l'élément piézoélectrique.

13. Dispositif gonflable actionné par fluide implantable (100) selon l'une quelconque des revendications 1 à 12, dans lequel le système de commande électronique (550) inclut une carte de circuit imprimé (520) qui inclut un processeur configuré pour :
recevoir des mesures de niveau de pression en provenance de l'au moins un dispositif de détection de pression ;
appliquer un algorithme de commande basé sur les mesures de niveau de pression reçues ; et
commander le fonctionnement de l'au moins une soupape et l'au moins une pompe conformément à l'algorithme de commande appliqué.

14. Dispositif gonflable actionné par fluide implantable (100) selon l'une quelconque des revendications 1 à 13, dans lequel le dispositif gonflable actionné par fluide implantable (100) est un sphincter urinaire artificiel (100A) ou une prothèse pénienne gonflable (100B).
